# EUROPEAN PATENT SPECIFICATION

(11) **EP 2 012 826 B1**
(45) Date of publication and mention of the grant of the patent: **13.04.2016**
(21) Application number: 07724892.0
(22) Date of filing: 04.05.2007
(51) Int. Cl.: A61K 45/06, A61K 39/395, A61K 38/49, A61P 27/02

(54) **COMBINATION COMPRISING A VEGF INHIBITOR AND A SERINE PROTEASE FOR TREATING NEOVASCULAR DISEASES**
KOMBINATION AUS EINEM VEGF-HEMMER UND EINER SERINPROTEASE ZUR BEHANDLUNG VON NEOVASKULÄREN ERKRANKUNGEN
COMBINAISON COMPRENANT UN INHIBITEUR DE VEGF ET UNE PROTEASE A SERINE POUR LE TRAITEMENT DES MALADIES NÉOVASCULAIRES

(30) Priority: 04.05.2006 EP 06360017
(43) Date of publication of application: 14.01.2009
(73) Proprietor: Fovea Pharmaceuticals, 75012 Paris (FR); Fondation Ophthalmologique Adolphe De Rothschild, 75940 Paris Cedex 19 (FR)
(72) Inventor: BONNEL, Sebastien, F-75005 Paris (FR); SAHEL, José, 75013 Paris (FR)
(74) Representative: Vossius & Partner Patentanwälte Rechtsanwälte mbB
(86) International application number: PCT/EP2007/003967
(87) International publication number: WO 2007/128526

(56) References cited:
- WO-A-2005/027973
- HOOPER CLAIRE Y ET AL: "New treatments in age-related macular degeneration." CLINICAL & EXPERIMENTAL OPHTHALMOLOGY OCT 2003, vol. 31, no. 5, October 2003 (2003-10), pages 376-391, XP002463484 ISSN: 1442-6404

## Description

The present application discloses compositions, kits of part and methods for inhibiting unwanted angiogenesis, including that of ocular tissues. More specifically, it discloses compositions, kits of part and methods for treating neovascularization, including that of ocular tissues, using agents that inhibit VEGF in combination with a second therapy.

The present invention provides the combination product, the pharmaceutical combination product and their use in the treatment of neovascular diseases of the ocular tissue as claimed in claims 1 to 4 attached hereto.

Angiogenesis, also called neovascularization, is a fundamental process whereby new blood vessels are formed. Under normal physiological conditions angiogenesis is highly regulated and essential for reproduction, embryonic development, tissue repair and wound healing (for a review see Carmeliet, 2005, Nature, 438, 932-936). However angiogenesis also occurs under various pathological conditions, including tumor growth and metastasis, inflammatory disorders such as rheumatoid arthritis, psoriasis, osteoarthritis, inflammatory bowel disease, Crohn's disease, ulcerative colitis and others, and ocular neovascularization such as in diabetic retinopathy, age related macular degeneration (AMD) and various other eye diseases (see for example Folkman, 1995, Nat. Med., 1, 27-31). Actually, angiogenesis occurs in response to various proangiogenic stimuli like growth factors, cytokines and other physiological molecules as well as other factors like hypoxia and low pH (Folkman and Shing, 1992, JBC, 267, 10931). The angiogenic cascade for development of new blood vessels requires the cooperation of a variety of molecules that regulate necessary cellular processes such as extracellular matrix (ECM) remodelling, invasion, migration, proliferation, differentiation and tube formation (Brooks, 1996, Eur. J. Cancer, 32A, 2423). After an initiation phase proangiogenic molecules like VEGF, bFGF, PDGF and others activate endothelial cells via stimulation of their cell surface receptors (for example VEGFR1/Flt-1 and VEGFR2/Flk-1/KDR; reviewed in Ferrara, 2004, Endocr. Rev., 25,581-611). These activated cells undergo a process of cellular proliferation, elevated expression of cell adhesion molecules, increased secretion of proteolytic enzymes and increased cellular migration and invasion. A number of distinct molecules are involved to promote proliferation and invasion, including members of the integrin, selectin and immunoglobulin gene super family for adhesion as well as proteolytic enzymes such as matrix metalloproteinases and serine proteinases for degrading the extracellular matrix (Brooks, 1996, Eur. J. Cancer, 32A, 2423). Finally, a complex cascade of biochemical signals derived from cell surface receptors interacting with extracellular matrix components and soluble factors, leading to lumen formation and differentiation into mature blood vessels.

While little is known about the molecular mechanisms of choroidal and/or retinal neovascularization, it has been shown that said specific angiogenic processes are responsible for the majority of severe vision loss in patients with AMD, as well as patients suffering from other retinopathies, such as diabetic retinopathy or retinopathy of prematurity.

Age-related macular degeneration is the leading cause of blindness in developed countries with approximately 15 million people with the disease in the United States. AMD is characterized as a progressive degenerative disease of the macula. There are two forms of AMD: neovascular and non-neovascular. The non-neovascular form of AMD is more common and leads to a slow deterioration of the macula with a gradual loss of vision over a period of years. The neovascular form of the disease is responsible for the majority of cases of severe vision loss and is due to proliferation of abnormal blood vessels behind the retina leading to hemorrhage and fibrosis which result in visual abnormalities. Current therapeutic efforts and clinical trials are primarily aimed at halting the growth of the neovascular membrane in AMD, e.g. using angiogenesis inhibitors, laser photocoagulation and/or photodynamic therapy (PDT) (see for example WO2004034889). However, only a fraction of eyes meet to the eligibility criteria for such therapeutic interventions and those treated have a high recurrence rate and low therapeutic benefit. Therefore, despite advances in treatment, AMD is still the most common cause of visual impairment in the developed world.

Another leading cause of blindness in adults between the ages of 20 and 74 years is diabetic retinopathy (DR). Seven million people in the United States have diabetes. While management of diabetic retinopathy has improved as a result of landmark clinical trials, risk of complications, such as loss of visual acuity, loss of night vision and loss of peripheral vision, remains significant and treatment sometimes fails. Diabetic retinopathy is characterized by aberrant neovascularization of the retinal vasculature with edema and breakdown in the blood-retinal barrier (BRB) that leads to hemorrhage, macular oedema, tissue damage and retinal scarring. Unfortunately, current treatment options (e.g. laser photocoagulation) are not fully satisfactory and the disease is often progressive.

An increasing body of evidence indicates that inhibition of different molecules involved in the angiogenic cascade offers the potential to treat probable cause of these neovascularization related disorders, including that of tumoral and ocular tissues, by blocking key mediating steps in disease progression (see for example Shibuya, 2003, Nippon Yakurigaku Zasshi, 122, 498-503 ; Ferrara, 2004, Endocrine Reviews, 25, 581-611; or US 20060030529). Example of these angiogenic inhibitors, including inhibitors of their related receptor, are known in the art and include, e.g., ZD6474 (Tuccillo et al., 2005, Clin Cancer Res., 11, 1268-1276); soluble Tie2 and VEGF-1 receptors (Hangai et al., 2001, Hum Gene Ther., 12, 1311-1321 and Honda et al., 2000, Gene Ther., 7, 978-985, respectively), angiopoietin (especially Ang-2) and PGDF inhibitors; pigment epithelium- derived factor (PEDF) (Rasmussen et al., 2001, Hum Gene Ther., 12, 2029-2032), endostatin (Mori et al., 2001, Am J Pathol., 159, 313-320), and angiostatin (Lai et al., 2001, Invest Ophthalmol Vis Sci., 42, 2401-2407) ; tissue inhibitor of metalloprotease-3 (Takahashi et al., 2000, Am J Ophthalmol., 130, 774-781); VEGF inhibitory aptamers, e.g., Macugen® (pegaptanib, Pfizer); antibodies or fragments thereof, e.g., anti-VEGF antibodies, e.g., bevacizumab (Avastin®, Genentech), or fragments thereof, e.g., ranibizumab (Lucentis®, Genetech); soluble fins-like tyrosine kinase 1 (sFlt1) polypeptides or polynucleotides (Harris et al., Clin Cancer Res. 2001 July; 7(7):1992-7; U.S. Pat. No. 5,861, 484); PTK/ZK which inhibits VEGF signal transduction by blocking the tyrosine kinase (Maier et al., 2005, Graefes Arch. Clin. Exp. Ophthalmol., 243, 593-600); KRN633 (Nakamura et al., 2004, Mol Cancer Ther., 3, 1639-1649); inhibitors of integrins (for example ανβ3 and α5β1); VEGF-Trap@ (Regeneron); intravitreal steroids, e.g., triamcinolone or anecortave acetate; and Alpha2-antiplasmin (Matsuno et al, Blood 2003; 120:3621-3628). Most of these angiogenic inhibitors are directed towards blocking the initial growth factor mediated activation step induced by vascular endothelial growth factor (VEGF). Therefore, VEGF has been considered as an appealing target for anticancer therapeutics, especially in combination with chemotherapy, radiotherapy or other antiangiogenic agents (see Ferrara, 2005, Oncology, 69, 11-16). Similarly, studies have shown regression or prevention of neovascularization in multiple vascular beds in several animal models, using various types of anti-VEGF agents (e.g. Gragoudas et al., 2004, N. Engl. J. Med., 351, 2805-2816 ; Rothen et al., 2005, Ophthalmol Clin North Am. , 18, 561-567 or Ng et al., 2006, Nat Rev Drug Discov., 5, 123-132) indicating that anti-VEGF therapy is a promising treatment for retinal and/or choroidal neovascularisation related disorders, such as CNV, AMD, diabetic retinopathy (for reviews of VEGF and its inhibitors, see, e.g., Campochiaro, 2004, Expert Opin Biol Ther., 4, 1395-1402; Ferrara, 2004, Endocr. Rev., 25, 581-611; and Verheul and Pinedo, 2003, Drugs Today, 39 Suppl C:81-93).

However, while these results are very encouraging, there is currently no standard and effective therapy for the treatment of neovascularisation and excessive vascular permeability in most patients, including that of ocular tissues. Actually, the interest of anti-angiogenic therapy for cancer by inhibition of the vascular endothelial growth factor (VEGF) pathway has been minored by occurrence of resistance to anti-VEGF treatment. This resistance appears to be associated with remodeled vasculature and to vessel stabilization (Ferrara and Kerbel, 2005, Nature, 438, 967-974).

Accordingly, existing methods for treating neovascular disease, including that of ocular tissues, are in need of improvement in their ability to inhibit or eliminate various forms of neovascularization, including retinal and/or choroidal neovascularization, and to treat related disorders. Future efforts must be directed towards the identification of new therapies in order to improve the efficacy of antiangiogenic therapy. The present invention fulfils these needs and further provides other related advantages.

The present invention intends to provide improved compositions for use in the treatment of neovascularisation of ocular tissues, using compounds that inhibit VEGF in combination with a thrombolytic compound. In one aspect of the present invention, there is provided compositions for use in preventing and treating choroidal and/or retinal neovascularization and related ophthalmic disorders, and more specifically AMD, CNV, retinopathy of prematurity, traumatic eye injury, diabetic retinopathy, inflammatory ophthalmic disorders (e.g. Birdshot retinochoroidopathy or multifocal choroiditis) and the like.

According to one embodiment, the Invention provides a combination product comprising a therapeutically effective amount of (i) at least one compound that inhibits VEGF and (ii) at least one thrombolytic compound, for simultaneous or consecutive administration, or administration which is staggered over time, in accordance with claim 1 attached hereto, wherein the compound that inhibits VEGF is bevacizumab and the thrombolytic compound is tissue plasminogen activator.

This combination product of the Invention is of particular interest for use in treating pathologies associated with neovascularization of ocular tissues.

According to one specific embodiment, said combination product further comprises an ophthalmically compatible solvent component.

The present invention is based on the surprising discovery that agent that results in the enhanced degradation of excess accumulated matrix, and more particularly thrombolytic agents, such as for example serine proteases able to digest the fibrin matrix, can successfully be employed in combination with one or more VEGF inhibitors, such as an anti-VEGF antibody or fragment thereof, to prevent and to treat neovascularization, more specifically choroidal and/or retinal neovascularization, and related disorders.

As used herein throughout the entire application, the terms "a" and "an" are used in the sense that they mean "at least one", "at least a first", "one or more" or "a plurality" of the referenced compounds or steps, unless the context dictates otherwise. More specifically, "at least one" and "one or more" means a number which is one or greater than one, with a special preference for one, two or three.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

As used herein, the term "comprising", "containing" when used to define products, compositions and methods, is intended to mean that the products, compositions and methods include the referenced compounds or steps, but not excluding others.

As used herein, the term "treatment" or "treating" encompasses prophylaxis and/or therapy. Accordingly the compositions and methods of the present invention are not limited to therapeutic applications and can be used in prophylaxis ones. Therefore "treating" or "treatment" of a state, disorder or condition includes: (i) preventing or delaying the appearance of clinical symptoms of the state, disorder or condition developing in a subject that may be afflicted with or predisposed to the state, disorder or condition but does not yet experience or display clinical or subclinical symptoms of the state, disorder or condition, (ii) inhibiting the state, disorder or condition, i.e., arresting or reducing the development of the disease or at least one clinical or subclinical symptom thereof, or (iii) relieving the disease, i.e. causing regression of the state, disorder or condition or at least one of its clinical or subclinical symptoms.

As used herein, "compound" refers to any agent, chemical substance, or substrate, whether organic or inorganic, or any protein including antibodies and functional fragments thereof, peptides, polypeptides, peptoids, nucleic acids, oligonucleotides, and the like.

As used herein, "compound that inhibits VEGF" refers to a compound that inhibits the activity or production of vascular endothelial growth factor (VEGF). It refers for example to compounds capable of binding VEGF, including small organic molecules, antibodies or antibody fragments specific to VEGF, peptides, cyclic peptides, nucleic acids, antisense nucleic acids, RNAi, and ribozymes that inhibit VEGF expression at the nucleic acid level. Examples of compounds that inhibits VEGF are nucleic acid ligands of VEGF, such as those described in US 6,168,778 or US 6,147,204, EYE001 (previously referred to as NX1838) which is a modified, pegylated aptamer that binds with high affinity to the major soluble human VEGF isoform ; VEGF polypepides (e.g. US 6,270,933 and WO 99/47677); oligonucleotides that inhibit VEGF expression at the nucleic acid level, for example antisense RNAs (e.g. US 5,710,136; US 5,661,135; US 5,641,756; US 5,639,872; and US 5,639,736). Other examples of inhibitors of VEGF signaling known in the art (see introduction of the present invention) include, e.g., ZD6474 (Tuccillo et al., 2005, Clin Cancer Res., 11, 1268-76); COX-2, Tie2 receptor, angiopoietin, and neuropilin inhibitors; pigment epithelium- derived factor (PEDF), endostatin, and angiostatin, soluble fins-like tyrosine kinase 1 (sFlt1) polypeptides or polynucleotides (Harris et al., 2001, Clin Cancer Res. , 7, 1992-1997; US 5,861, 484); PTK787/ZK222 584; KRN633 (Maier et al., 2004, Mol Cancer Ther., 3, 1639-1649); VEGF-Trap® (Regeneron); intravitreal steroids, e.g., triamcinolone; and Alpha2-antiplasmin (Matsuno et al, 2003, Blood, 120, 3621-3628) . For reviews of VEGF and its inhibitors, see, e.g., Campochiaro, 2004, Expert Opin Biol Ther., 4, 1395-1402; Ferrara, 2004, Endocr. Rev., 25, 581-611). Compounds that inhibits VEGF are anti-VEGF antibodies or functional antibody fragments, most preferably humanized antibodies or functional fragment thereof, such as those described in US 6,100,071, US 5,730,977 or WO98/45331. Compounds that inhibit VEGF are antibodies to, or antibody fragments thereof, or aptamers of VEGF or a related family member such as (VEGF B. I C, D; PDGF). Examples are anti-VEGF antibodies, e.g Avastin™ (also reviewed as bevacizumab, Genentech), or fragments thereof, e.g. Lucentis™ (also reviewed as rhuFAb V2 or AMD-Fab ; ranibizumab, Genentech), and other anti-VEGF compounds such as VEGF inhibitory aptamers, e.g., Macugen™ (also reviewed as pegaptanib sodium, anti-VEGF aptamer or EYE001, Pfizer). According to the present invention, the compound that inhibits VEGF is bevacizumab.

As used herein, "antibody" encompasses polyclonal and monoclonal antibody preparations, CDR-grafted antibody preparations, as well as preparations including hybrid antibodies, altered antibodies, F(AB)'₂ fragments, F(AB) molecules, Fv fragments, single domain antibodies, chimeric antibodies and functional fragments thereof which exhibit immunological binding properties of the parent antibody molecule. The antibodies can also be humanized. The term "monoclonal antibody" is not limited to antibodies produced through hybridoma technology. The term "monoclonal antibody" refers to an antibody or functional fragment thereof that is derived from a single clone, including any eukaryotic, prokaryotic, or phage clone, and not the method by which it is produced.

As used herein, "thrombolytic compound" refers to a compound, and more particularly to a protease, that is able directly or indirectly to activate digestion of the fibrinogen and/or fibrin matrix. These thrombolytic compounds are well known in the art as agents for dissolving an occlusive artery or vein thrombus and for cardiovascular diseases treatment. The thrombolytic compound of the Invention is tissue plasminogen activator [tPA ; including reteplase (Rapilysine™), alteplase (Actilyse™), Tenecteplase (Metalyse™)].

According to another embodiment, said combination product further comprises a biocompatible polymeric or fibrin glue component in an amount effective to delay release of the said compound that inhibits VEGF and/or said thrombolytic compound, especially into the interior of the eye after the combination product is intraocularly placed in the eye. According to another specific embodiment, said combination product further comprises an ophthalmically compatible solvent component in an amount effective to solubilize the said polymeric or fibrin glue component, the combination product being effective, after being intraocularly placed into the interior of the eye, to form a delayed release of the said compound that inhibits VEGF and/or said thrombolytic compound in the eye relative to intraocular placement of a substantially identical composition without the polymeric or fibrin glue component.

In another aspect of the invention, the combination product of the invention may further comprise a compound selected in the group consisting of a glucocorticoid (e.g. prednisolone, prednisone, dexamethasone, triamcinolone), an oestrogen (e.g. oestrodiol), an androgen (e.g. testosterone) retinoic acid derivatives (e. g. 9-cis-retinoic acid, 13-trans-retinoic acid, all-trans retinoic acid), a vitamin D derivative (e. g. calcipotriol, calcipotriene), a non-steroidal anti-inflammatory agent, a non-steroidal immunophilin-dependent immunosuppressant (NsIDI - e.g. cyclosporin, tacrolimus), a selective serotonin reuptake inhibitor (SSR1 ; e.g. fluoxetine, sertraline), a tricyclic antidepressant (TCA ; e.g. maprotiline, amoxapine), a phenoxy phenol (e.g. triclosan), an antihistaminine (e.g. loratadine, epinastine), a phosphodiesterase inhibitor (e.g. ibudilast), a vitamin D derivative, an anti-infective agent, a protein kinase C inhibitor, a MAP kinase inhibitor, an anti-apoptotic agent, a growth factor, a nutrient vitamin, an unsaturated fatty acid, and/or ocular anti-infective agents, for the treatment of the ophthalmic disorders set forth herein (see for example compounds disclosed in US 2003/0119786; WO 2004/073614 ; WO 2005/051293 ; US 2004/0220153 ; WO 2005/027839 ; WO 2005/037203 ; WO 03/0060026). In still other embodiments of the invention, a mixture of these agents may be used. Ocular anti-infective agents that may be used include, but are not limited to penicillins (ampicillin, aziocillin, carbenicillin, dicloxacillin, methicillin, nafcillin, oxacillin, penicillin G, piperacillin, and ticarcillin), cephalosporins (cefamandole, cefazolin, cefotaxime, cefsulodin, ceftazidime, ceftriaxone, cephalothin, and moxalactam), aminoglycosides (amikacin, gentamicin, netilmicin, tobramycin, and neomycin), miscellaneous agents such as aztreonam, bacitracin, ciprofloxacin, clindamycin, chloramphenicol, cotrimoxazole, fusidic acid, imipenem, metronidazole, teicoplanin, and vancomycin), antifungals (amphotericin B, clotrimazole, econazole, fluconazole, flucytosine, itraconazole, ketoconazole, miconazole, natamycin, oxiconazole, and terconazole), antivirals (acyclovir, ethyldeoxyuridine, foscarnet, ganciclovir, idoxuridine, trifluridine, vidarabine, and (S)-1-(3-dydroxy-2-phospho-nyluethoxypropyl) cytosine (HPMPC)), antineoplastic agents (cell cycle (phase) nonspecific agents such as alkylating agents (chlorambucil, cyclophosphamide, mechlorethamine, melphalan, and busulfan), anthracycline antibiotics (doxorubicin, daunomycin, and dactinomycin), cisplatin, and nitrosoureas), antimetabolites such as antipyrimidines (cytarabine, fluorouracil and azacytidine), antifolates (methotrexate), antipurines (mercaptopurine and thioguanine), bleomycin, vinca alkaloids (vincrisine and vinblastine), podophylotoxins (etoposide (VP-16)), and nitrosoureas (carmustine, (BCNU)), immunosuppressant agents such as cyclosporin A and SK506, and anti-inflammatory or suppressive agents (inhibitors), and inhibitors of proteolytic enzymes such as plasminogen activator inhibitors. Doses for topical and sub-conjunctival administration of the above agents, as well as intravitreal dose and vitreous half-life may be found in Intravitreal Surgery Principles and Practice, Peyman G A and Shulman, J Eds., 2nd edition, 1994, Appleton- Longe, the relevant sections of which are expressly incorporated by reference herein.

According to another embodiment, said combination product further comprises a pharmaceutically acceptable carrier. Such pharmaceutical carriers can be sterile liquids, such as water and oils, including those of petroleum, animal, vegetable or synthetic origin, such as peanut oil, soybean oil, mineral oil, and the like. Saline solutions and aqueous dextrose, polyethylene glycol (PEG) and glycerol solutions can also be employed as liquid carriers, particularly for injectable solutions. Suitable pharmaceutical excipients include starch, glucose, lactose, sucrose, gelatin, malt, rice, sodium stearate, glycerol monostearate, glycerol, propylene, glycol, water, and the like. The combination product, if desired, can also contain minor amounts of wetting or emulsifying agents, or pH buffering agents. Examples of suitable pharmaceutical carriers are described in "Remington's Pharmaceutical Sciences" by E. W. Martin. In a preferred embodiment, the combination product is formulated in accordance with routine procedures as a pharmaceutical composition adapted for injection into the eye. Typically, combination products for injection are solutions in sterile isotonic aqueous buffer. Where necessary, the combination product may also include a solubilizing agent. Generally, the ingredients are supplied either separately or mixed together in unit dosage form, for example, as a dry lyophilized powder or water free concentrate in a hermetically sealed container such as an ampoule or sachet indicating the quantity of active agent. Where the combination product is to be administered by infusion, it can be dispensed with an infusion bottle containing sterile pharmaceutical grade water or saline. Where the combination product is administered by injection, an ampoule of sterile water for injection or saline can be provided so that the ingredients may be mixed prior to administration.

The present application also discloses a method for inhibiting, treating, or preventing neovascularization, including that of ocular tissues, and related disease or condition, in a patient in need of such treatment that comprises the step of administering a combination product of the present invention in said patient.

The administration of (i) at least one compound that inhibits VEGF and (ii) at least one thrombolytic compound results in a synergistic effect for inhibiting, treating, or preventing the neovascularization of ocular tissues, and related disorders.

As used herein, "patient" is meant any animal having ocular tissue that may be subject to neovascularization. Preferably, the animal is a vertebrate, particularly a member of the mammalian species and includes, but is not limited to, domestic animals (e.g. cows, hogs, sheep, horses, dogs, and cats), primates including humans. The term "patient" is in no way limited to a special disease status, it encompasses both patients who have already developed a disease of interest and patients who are not sick.

According to the present invention, the combination product can be used to inhibit, to prevent and to treat a number of diseases and disorders marked by the development of ophthalmic neovascularization. According to the present invention, the ophthalmic neovascularization and related disorders are preferably macular edema, ischemic retinopathy, intraocular neovascularization, age-related macular degeneration, corneal neovascularization, retinal neovascularization, choroidal neovascularization, retinopathy of prematurity, traumatic eye injury, diabetic macular edema, diabetic retina ischemia, diabetic retinal oedema, proliferative diabetic retinopathy, birdshot disease, multifocal choroiditis and any neovascularization associated with any pathological condition of the eye.

According to the present invention, the administration of (i) at least one compound that inhibits VEGF and (ii) at least one agent that results in the enhanced degradation of excess accumulated matrix, i.e. at least one thrombolytic compound, of the combination product can be simultaneous or consecutive administration, or administration which is staggered over time. Simultaneously refers to a coadministration. In this case, these two essential compounds [(i) and (ii)] can be mixed to form a composition prior to being administered, or can be administered as separate compounds at the same time to the patient. It is also possible to administer them consecutively, that is to say one after the other, irrespective of which component of the combination product according to the invention is administered first. Finally, it is possible to use a mode of administration which is staggered over time or is intermittent and which stops and restarts at intervals which may or may not be regular. It is pointed out that the routes and sites of administration of the two components can be different. The time interval between the administrations is not critical and can be defined by the skilled person. It is possible to recommend an interval of from 10 min to 72 h, advantageously of from 30 min to 48 h, preferably of from 1 to 24 h and, very preferably, of from 1 to 6 h; but the interval can be larger, and being over month.

Administration of the combination product for ophthalmic applications is preferably by intraocular injection, although other modes of administration may be effective. Typically, ophthalmic composition will be delivered intraocularly (by chemical delivery system or invasive device) to an individual. However, the invention is not limited to intraocular delivery in that it also includes topically (extraocular application) or systemically (e.g. oral or other parenteral route such as for example subcutaneous administration) or intratumoral administration. Parenteral administration is used in appropriate circumstances apparent to the practitioner. Preferably, the ophthalmic compositions are administered in unit dosage forms suitable for single administration of precise dosage amounts.

As mentioned above, delivery to areas within the eye, *in situ* can be accomplished by injection, cannula or other invasive device designed to introduce precisely metered amounts of a desired ophthalmic composition to a particular compartment or tissue within the eye (e.g. posterior chamber or retina). An intraocular injection may be into the vitreous (intravitreal), or under the conjunctiva (subconjunctival), or behind the eye (retrobulbar), into the sclera, or under the Capsule of Tenon (sub- Tenon), and may be in a depot form. Other intraocular routes of administration and injection sites and forms are also contemplated and are within the scope of the invention. In prefered embodiment the combination product of the invention will be delivered by sub-retinal injection.

In one embodiment, the ophthalmic composition is intraocularly injected (e.g., into the vitreous or sub retinal) to treat or prevent an ophthalmic condition. When administering the ophthalmic composition by intraocularly injection, the active agents should be concentrated to minimise the volume for injection. Preferably, the volume for injection is less than about 5 ml. Volumes such as this may require compensatory drainage of the vitreous fluid to prevent increases in intraocular pressure and leakage of the injected fluid through the opening formed by the delivery needle. More preferably, the volume injected is between about 1.0 ml and 0.05 ml. Most preferably, the volume for injection is approximately 0.1 ml.

For injection, a concentration less than about 20 mg/ml may be injected, and any amount may be effective depending upon the factors previously described. Preferably a dose of about 10 mg/ml is administered. Sample concentrations include, but are not limited to, about 5 µg/ml to about 50 µg/ml; about 25 µg/ml to about 100 µg/ml; about 100 µg/ml to about 200 µg/ml; about 200 µg/ml to about 500 µg/ml; about 500 µg/ml to about 750 µg/ml; about 500 µg/ml up to 1 mg/ml etc. preferred 50mg/ml. The concentration of compound (i) and (ii) can further be different for one said combination product. In preferred embodiment, a maximum of 100 micrograms of compound (ii) is administered. And in another preferred embodiment, only 0.1 ml of the composition containing compound (i) at 10 mg/ml is administered to the patient.

Intraocular injection may be achieved by a variety of methods well known in the art. For example, the eye may be washed with a sterilising agent such as Betadine® and the compound of the Invention is injected in an appropriate carrier with a fine gauge needle (eg 27 gauge) at a position in the eye such that the compound will settle to the posterior pole towards the ventral surface. It may be necessary to prepare the eye for injection by application of positive pressure prior to injection. In some cases, preliminary vitrectomy may be necessary. Local anaesthetic or general anaesthetic may be necessary.

The syringe used in practicing the method of this invention is suitably one which can accommodate a 21 to 40 gauge needle and is preferably of a small volume, for example 1.5 ml, or more preferably 0.1 ml. Although it is possible that the needle and syringe may be of the type where the needle is removable from the syringe, it is preferred that the arrangement is of a unitary syringe/needle construction. This would clearly limit the possibility of disengagement of the needle from the syringe. It is also preferred that the arrangement be tamper evident. The combination product of the present invention may therefore be provided in the form of a single unit dose, or separated unit doses each containing part of the combination product, in a pre-prepared syringe ready for administration.

A suitable style of syringe is, for example, sold under the name of Uniject® manufactured by Becton Dickinson and Company. In this style of syringe, the material is expelled through the needle into the eye by pressure applied to the sides of a pliable reservoir supplying the needle, rather than by a plunger. As the name implies, the construction of the reservoir and needle forms a single unit.

Topical application of ophthalmic combination product of the invention for the treatment or prevention of ophthalmic disorders may be as ointment, gel or eye drops. The topical ophthalmic composition may further be an *in situ* gellable aqueous formulation. Such a formulation comprises a gelling agent in a concentration effective to promote gelling upon contact with the eye or with lacrimal fluid in the exterior of the eye. Suitable gelling agents include, but are not limited to, thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The phrase *"in situ* gellable" as used herein embraces not only liquids of low viscosity that form gels upon contact with the eye or with lacrimal fluid in the exterior of the eye, but also more viscous liquids such as semi-fluid and thixotropic gels that exhibit substantially increased viscosity or gel stiffness upon administration to the eye.

To prepare a topical ophthalmic composition for the treatment of ophthalmic disorders, a therapeutically effective amount of the combination product of the invention is placed in an ophthalmological vehicle as is known in the art. For example, topical ophthalmic formulations containing steroids are disclosed in US 5,041,434, whilst sustained release ophthalmic formulations of an ophthalmic drug and a high molecular weight polymer to form a highly viscous gel have been described in US 4,271,143 and US 4,407,792. Further GB 2007091 describes an ophthalmic composition in the form of a gel comprising an aqueous solution of a carboxyvinyl polymer, a water-soluble basic substance and an ophthalmic drug. Alternatively, US 4,615,697, discloses a controlled release composition and method of use based on a bioadhesive and a treating agent, such as an anti-inflammatory agent.

The amount of the combination product to be administered and the concentration of the compound in the topical ophthalmic combination product used in the method depend upon the selected diluent, delivery system or device, the clinical condition of the patient, the side effects and the stability of the compound in the formulation. Thus, the physician employs the appropriate preparation containing the appropriate concentration of the compounds (i) and/or (ii) and selects the amount of formulation administered, depending upon clinical experience with the patient in question or with similar patients.

As the combination product contains two or more active agents, the active agents may be administered as a mixture, as an admixture, in the same ophthalmic composition, in separate formulations, in extended release formulations, liposomes, microcapsules, or any of the previously described embodiments.

The combination product may be also administered as a slow release formulation, with a carrier formulation such as microspheres, microcapsules, liposomes, etc., as a topical ointment or solution, an intravenous solution or suspension, or in an intraocular injection, as known to one skilled in the art to treat or prevent ophthalmic disorders.

A time-release drug delivery system may be administered intraocularly to result in sustained release of the combination product over a period of time. The combination product may be in the form of a vehicle, such as a micro- or macro-capsule or matrix of biocompatible polymers such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in US Patents Nos. 6,667,371; 6,613,355; 6,596,296; 6,413,536; 5,968,543; 4,079, 038; 4,093,709; 4,131,648; 4,138,344; 4,180,646; 4,304,767; 4,946,931, each of which is expressly incorporated by reference herein in its entirety, or lipids that may be formulated as microspheres or liposomes. A microscopic or macroscopic ophthalmic composition may be administered through a needle, or may be implanted by suturing within the eye, eg intravitreal cavity or sub-retinal space. Delayed or extended release properties may be provided through various formulations of the vehicle (coated or uncoated microsphere, coated or uncoated capsule, lipid or polymer components, unilamellar or multilamellar structure, and combinations of the above, etc.). The formulation and loading of microspheres, microcapsules, liposomes, etc and their ocular implantation are standard techniques known by one skilled in the art.

The invention also provides a combination product for use in the treatment or prophylaxis of ophthalmic disorders related to neovascularisation in a biocompatible, biodegradable matrix, for example in the form of a gel or polymer which is preferably suited for insertion into the retina or into a cavity of the eye, anterior or posterior, as an implant. In the case that the combination product is delivered as an implant, it may be incorporated in any known biocompatible biodegradable matrix as a liquid, or in the form, for example, of a micelle using known chemistry or as microparticles.

Slow or extended-release delivery systems include any of a number of biopolymers (biological-based systems), systems employing liposomes, colloids, resins, and other polymeric delivery systems or compartmentalized reservoirs, can be utilized with the compositions described herein to provide a continuous or long term source of therapeutic compound.

In any slow release device prepared, the said compounds (i) and/or (ii) are preferably present in an amount of about 10% to 90% by weight of the implant. More preferably, the said compounds (i) and/or (ii) are from about 50% to about 80% by weight of the implant. In a preferred embodiment, the said compounds (i) and/or (ii) are about 50% by weight of the implant. In a particularly preferred embodiment, the said compounds (i) and/or (ii) are about 70% by weight of the implant.

In one form, implants of the present invention are formulated with compounds (i) and/or (ii) entrapped within the bio-erodible polymer matrix. Release of the compounds is achieved by erosion of the polymer followed by exposure of previously entrapped compound to the vitreous, and subsequent dissolution and release of compound. The release kinetics achieved by this form of drug release are different than that achieved through formulations which release drug through polymer swelling, such as with hydrogels such as methylcellulose. In that case, the active compound is not released through polymer erosion, but through polymer swelling, which releases active compound as liquid diffuses through the pathways exposed. The parameters which determine the release kinetics include the size of the active compound particles, the water solubility of the active compound, the ratio of active compound to polymer, the method of manufacture, the surface area exposed, and the erosion rate of the polymer.

Exemplary biocompatible, non-biodegradable polymers of particular interest include polycarbamates or polyureas, particularly polyurethanes, polymers which may be cross-linked to produce non- biodegradable polymers such as cross-linked poly(vinyl acetate) and the like. Also of particular interest are ethylene-vinyl ester copolymers having an ester content of 4% to 80% such as ethylene-vinyl acetate (EVA) copolymer, ethylene-vinyl hexanoate copolymer, ethylene-vinyl propionate copolymer, ethylene-vinyl butyrate copolymer, ethylene-vinyl pentantoate copolymer, ethylene-vinyl trimethyl acetate copolymer, ethylene-vinyl diethyl acetate copolymer, ethylene-vinyl 3-methyl butanoate copolymer, ethylene-vinyl 3-3-dimethyl butanoate copolymer, and ethylene-vinyl benzoate copolymer.

Additional exemplary naturally occurring or synthetic non-biodegradable polymeric materials include poly(methylmethacrylate), poly(butylmethacrylate), plasticized poly(vinylchloride), plasticized poly(amides), plasticized nylon, plasticized soft nylon, plasticized poly(ethylene terephthalate), natural rubber, silicone, poly(isoprene), poly(isobutylene), poly(butadiene), poly(ethylene), poly(tetrafluoroethylene), poly(vinylidene chloride), poly(acrylonitrile, cross-linked poly(vinylpyrrolidone), poly(trifluorochloroethylene), chlorinated poly(ethylene), poly(4,4'- isopropylidene diphenylene carbonate), vinylidene chloride-acrylonitrile copolymer, vinyl chloridediethyl fumarate copolymer, silicone, silicone rubbers (especially the medical grade), poly(dimethylsiloxanes), ethylene- propylene rubber, silicone-carbonate copolymers, vinylidene chloride- vinyl chloride copolymer, vinyl chloride-acrylonitrile copolymer, vinylidene chloride-acrylonitrile copolymer, poly(olefins), poly(vinyl-olefins), poly(styrene), poly(halo-olefins), poly(vinyls), poly(acrylate), poly(methacrylate), poly(oxides), poly(esters), poly(amides), and poly(carbonates). Diffusion of the active compounds (i) and/or (ii) from the implant may also be controlled by the structure of the implant. For example, diffusion of the compounds (i) and/or (ii) from the implant may be controlled by means of a membrane affixed to the polymer layer comprising the drug. The membrane layer will be positioned intermediate to the polymer layer comprising the compounds (i) and/or (ii) and the desired site of therapy. The membrane may be composed of any of the biocompatible materials indicated above, the presence of agents in addition to the compounds (i) and/or (ii) present in the polymer, the composition of the polymer comprising the compounds (i) and/or (ii), the desired rate of diffusion and the like.

The skilled reader will appreciate that the duration over which any of the ophthalmic combination product used in the method of the invention will dwell in the ocular environment will depend, inter alia, on such factors as the physicochemical and/or pharmacological properties of the compounds employed in the formulation, the concentration of the compound employed, the bioavailability of the compound, the disease to be treated, the mode of administration and the preferred longevity of the treatment. Where that balance is struck will often depend on the longevity of the effect required in the eye and the ailment being treated.

The frequency of treatment is determined according to the disease being treated, the deliverable concentration of the compounds (i) and/or (ii) and the method of delivery. If delivering the combination product by intravitreal injection, the dosage frequency may be monthly. Preferably, the dosage frequency is every three months. The frequency of dosage may also be determined by observation, with the dosage being delivered when the previously delivered combination product is visibly cleared. In general, an effective amount of the compound is that which provides either subjective relief of symptoms or an objectively identifiable improvement as noted by the clinician or other qualified observer.

Ophthalmic combination product prepared for used in the method of the present invention to prevent or treat ophthalmic disorders will preferably have dwell times from hours to many months and possibly years, although the latter time period requires special delivery systems to attain such duration and/or alternatively requires repetitive administrations. Most preferably the combination product for use in the method of the invention will have a dwell time (ie duration in the eye) of hours (i.e. 1 to 24 hours), days (i.e. 1, 2, 3, 4, 5, 6 or 7 days) or weeks (i.e. 1, 2, 3, 4 weeks). Alternatively, the combination product will have a dwell time of at least a few months such as, 1 month, 2 months, 3 months, with dwell times of greater than 4, 5, 6, 7 to 12 months being achievable.

The combination products for use in the treatment or prophylaxis of ophthalmic conditions according to the present invention may be administered alone, or in combination with one or more other therapies such as photodynamic therapy, laser surgery, laser photocoagulation or one or more biological or pharmaceutical treatments. These therapies are well known from the skilled man in the art and widely disclosed in the litterature.

The present application discloses a method for inhibiting, treating, or preventing an angiogenesis-mediated ophthalmic disease or condition in a patient, comprising administering to said patient an amount effective to inhibit, reduce, or prevent angiogenesis of a combination product comprising (i) at least one compound that inhibits VEGF and (ii) at least one thrombolytic compound.

The present application discloses a method for inhibiting, treating, or preventing an angiogenesis-mediated ophthalmic disease or condition in a patient, comprising co-administering to said patient an amount effective to inhibit, reduce, or prevent angiogenesis of (i) at least one compound that inhibits VEGF and (ii) at least one thrombolytic compound.

The present application discloses a method to cause regression of neovascularization in a patient, comprising administering to said patient an amount effective of a combination product comprising (i) at least one compound that inhibits VEGF and (ii) at least one thrombolytic compound.

The present application discloses a method to cause regression of neovascularization in a patient, comprising co-administering to said patient of (i) at least one compound that inhibits VEGF and (ii) at least one thrombolytic compound.

As used herein, "to cause regression of neovascularization" means to decrease the amount of neovasculature, especially in the eye, in a subject afflicted with neovascular disease, especially an ocular neovascular disease as defined above.

Also disclosed is the use of (i) at least one compound that inhibits VEGF and (ii) at least one agent that results in the enhanced degradation of excess accumulated matrix for the preparation of a composition useful for the prophylactic or therapeutic treatment of neovascularization, of ocular tissues, and related disorders in a patient, and more specifically those cited above.

Further disclosed is the use of (i) at least one compound that inhibits VEGF and (ii) at least one thrombolytic compound for the preparation of a composition useful for the prophylactic or therapeutic treatment of neovascularization, of ocular tissues, and related disorders in a patient, and more specifically those cited above.

The invention described herein may include one or more range of values (eg size, concentration etc). A range of values will be understood to include all values within the range, including the values defining the range, and values adjacent to the range which lead to the same or substantially the same outcome as the values immediately adjacent to that value which defines the boundary to the range.

### Examples: Treatment of patients suffering from sub retinal neovascularization due to age related macular degeneration (AMD) with the combination product of the Invention.

### Figures legends :

Figure 1 : Occult neovascularisation in Patient 1 measured by fluorescein angiography before treatment with combination product of the Invention.
Figure 2 : Occult neovascularisation in Patient 1 measured by infracyanine angiography before treatment with combination product of the Invention.
Figure 3 : Occult neovascularisation in Patient 1 measured by fluorescein angiography after treatment with combination product of the Invention.
Figure 4 : Occult neovascularisation in Patient 1 measured by infracyanine angiography after treatment with combination product of the Invention.
Figure 5 : Occult neovascularisation in Patient 2 measured by fluorescein angiography before treatment with combination product of the Invention.
Figure 6 : Occult neovascularisation in Patient 2 measured by infracyanine angiography before treatment with combination product of the Invention.
Figure 7 : Occult neovascularisation in Patient 2 measured by fluorescein angiography after treatment with combination product of the Invention.
Figure 8 : Occult neovascularisation in Patient 2 measured by infracyanine angiography after treatment with combination product of the Invention.

Two patients (Patient 1 and Patient 2) suffering from sub retinal neovascularization due to age related macular degeneration (AMD) were treated with a co administration of two compounds according to the present invention : (i) a VEGF inhibitor (bevacizumab, Avastin™, Genentech), and (ii) a thrombolytic compound (recombinant tissue plasminogen activator: rTPA).

Both compounds were administrated locally at the same time into the sub retinal space after a pars plana vitrectomy procedure. A total amount of 1.25 mg of bevacizumab associated with 50 microg of rTPA into a total volume of 0.6 ml of buffer saline solution were injected into the sub retinal space. A SF6 gas tamponade or air tamponade was realized at the end of surgery associated with prone posturing during five days (beginning a day after the surgical procedure).

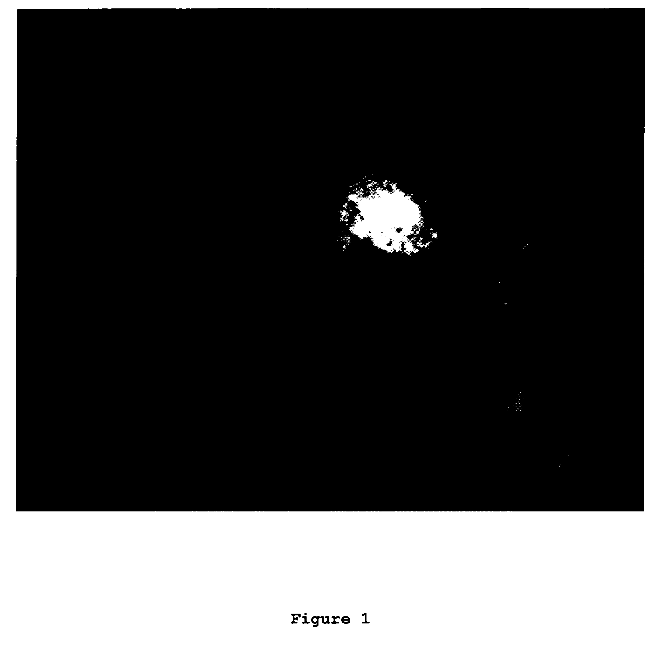

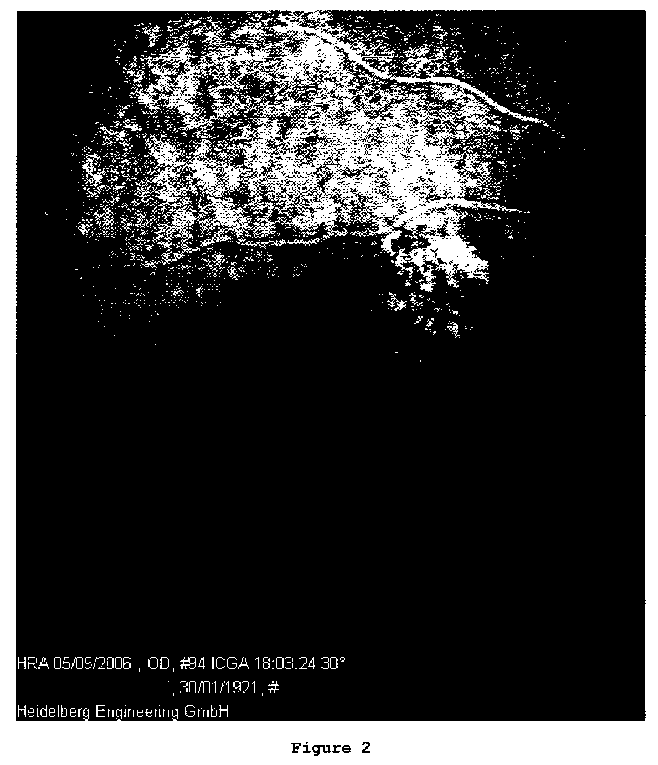

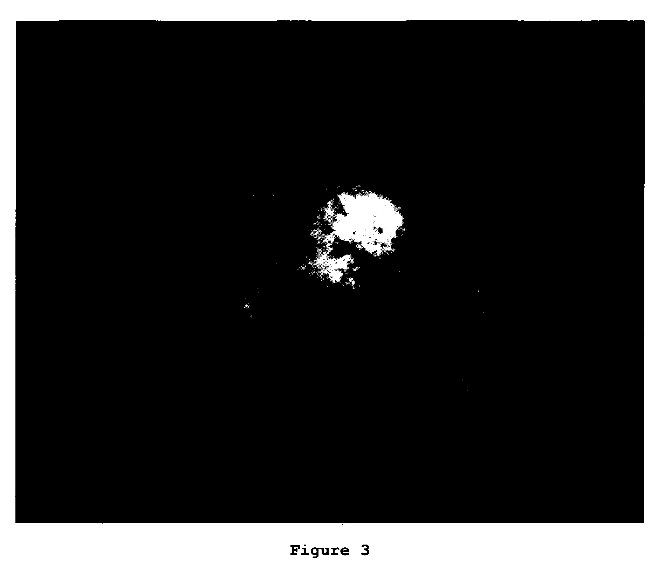

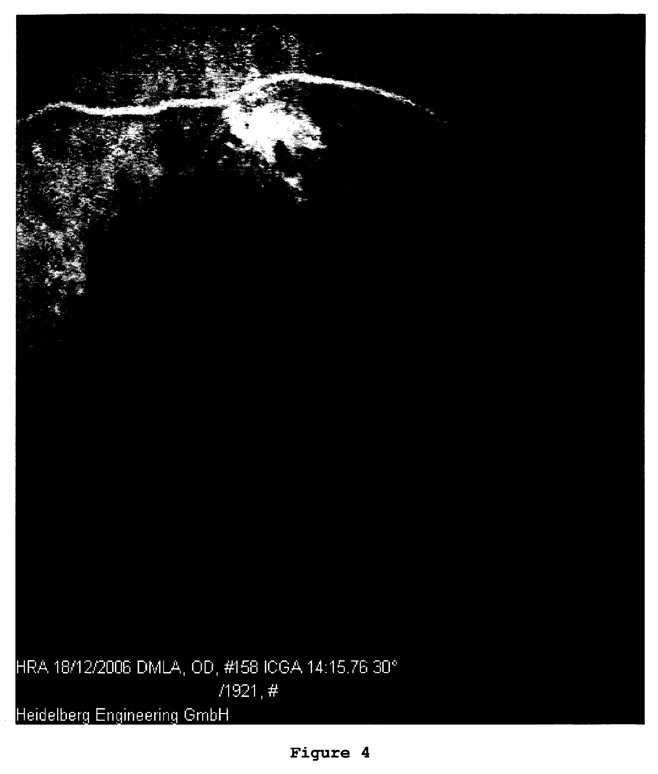

The first patient (Patient 1) was an 86 year old woman suffering from occult neovascularization in her right eye (see fig. 1 and 2). The neovascularization was associated with a pigment epithelium detachment (PED) and moderate sub retinal haemorrhage. The eye was pseudophakic. Before treatment according to the Invention, the best corrected EDTRS visual acuity was 20/250. Two months after treatment, visual acuity was 20/50 and the optical coherence tomography (OCT) showed complete regression of PED. The activity of the occult neovascularization had diminished on fluorescein and infracyanine angiography (see fig. 3 and 4).

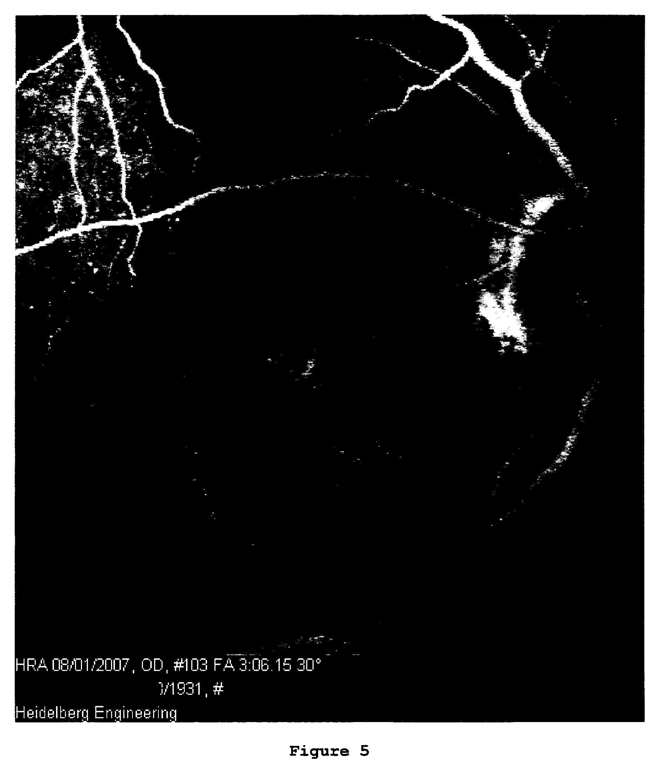

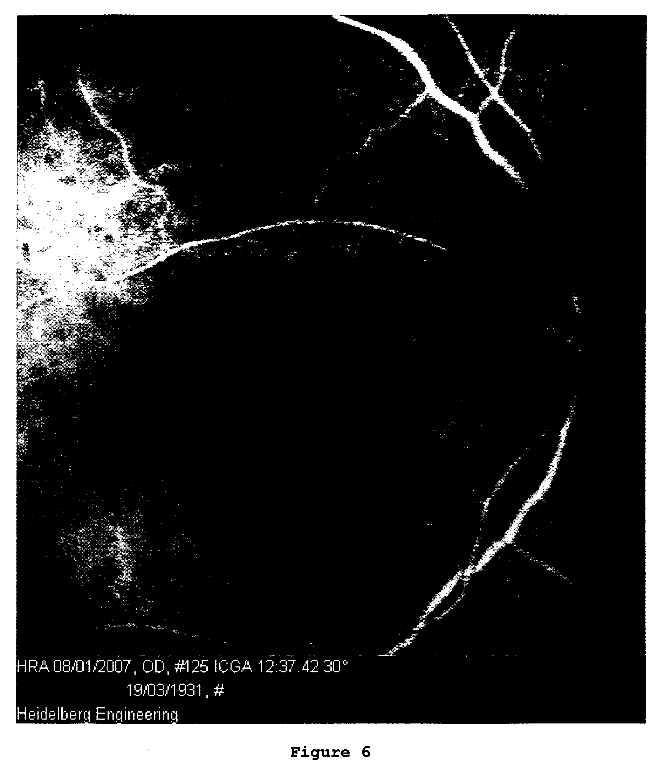

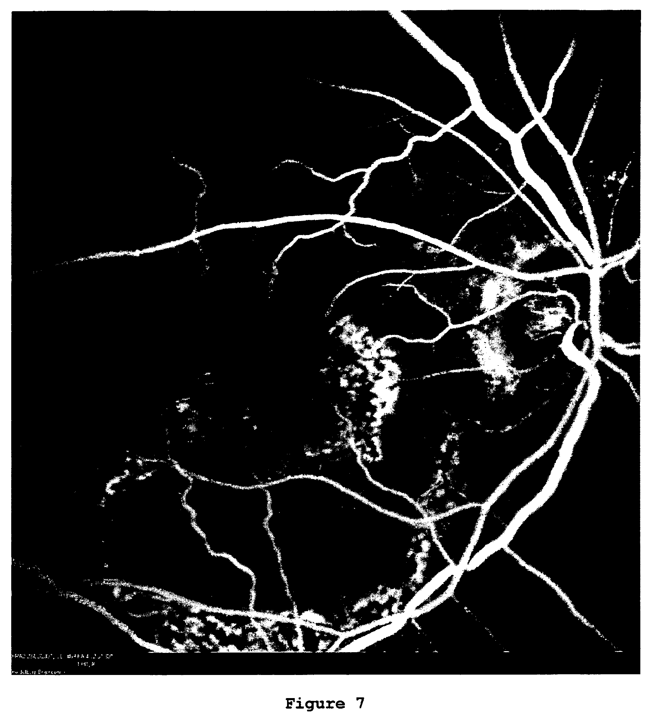

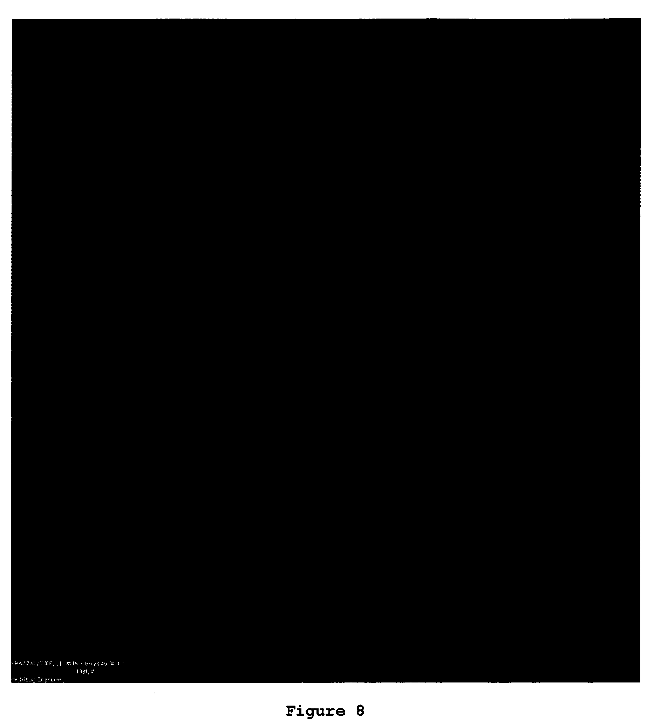

The second patient (Patient 2) was a 75 year old man suffering from classic neovascularization with little sub retinal haemorrhage in his right eye (see fig. 5 and 6). The eye was phakic. The best corrected EDTRS visual acuity improved from 20/120 before treatment of the Invention to 20/50 two months after said treatment. The subretinal space appeared anatomical normal on OCT and no persitant neovascularization was noted on fluorescein (Figure 7) and infracyanine angiography (Figure 8) two months after treatment.

## Claims

1. A combination product comprising a therapeutically effective amount of (i) at least one compound that inhibits VEGF and (ii) at least one thrombolytic compound, wherein the compound that inhibits VEGF is bevacizumab and the thrombolytic compound is tissue plasminogen activator (TPA).

2. A pharmaceutical combination product comprising a therapeutically effective amount of (i) at least one compound that inhibits VEGF and (ii) at least one thrombolytic compound, wherein the compound that inhibits VEGF is bevacizumab and the thrombolytic compound is tissue plasminogen activator (TPA).

3. The pharmaceutical combination product according to claim 2 for use in the treatment of neovascular diseases of the ocular tissue in a patient in need thereof.

4. Pharmaceutical combination product for use according to claim 3, wherein said neovascular disease is selected in the group consisting in macular edema, ischemic retinopathy, intraocular neovascularization, age-related macular degeneration, corneal neovascularization, retinal neovascularization, choroidal neovascularization, retinopathy of prematurity, traumatic eye injury, diabetic macular edema, diabetic retina ischemia, diabetic retinal oedema, proliferative diabetic retinopathy, birdshot disease, multifocal choroiditis and any neovascularization associated with any pathological condition of the eye.

## Patentansprüche

1. Kombinationsprodukt, umfassend eine therapeutisch wirksame Menge an (i) mindestens einer Verbindung, die VEGF inhibiert und (ii) mindestens einer thrombolytischen Verbindung, wobei die Verbindung, die VEGF inhibiert, Bevacizumab ist und die thrombolytische Verbindung gewebespezifischer Plasminogenaktivator (TPA) ist.

2. Pharmazeutisches Kombinationsprodukt, umfassend eine therapeutisch wirksame Menge an (i) mindestens einer Verbindung, die VEGF inhibiert und (ii) mindestens einer thrombolytischen Verbindung, wobei die Verbindung, die VEGF inhibiert, Bevacizumab ist und die thrombolytische Verbindung gewebespezifischer Plasminogenaktivator (TPA) ist.

3. Pharmazeutisches Kombinationsprodukt nach Anspruch 2 zur Verwendung bei der Behandlung von neovaskulären Erkrankungen des okularen Gewebes bei einem Patienten, der diese benötigt.

4. Pharmazeutisches Kombinationsprodukt zur Verwendung nach Anspruch 3, wobei die neovaskuläre Erkrankung ausgewählt ist aus der Gruppe bestehend aus Makulaödem, ischämischer Retinopathie, intraokularer Neovaskularisation, altersbedingter Makuladegeneration, kornealer Neovaskularisation, retinaler Neovaskularisation, choroidaler Neovaskularisation, Retinopathia praematurorum, traumatischer Augenverletzung, diabetischem Makulaödem, diabetischer Retinaischämie, diabetischem Retinaödem, proliferativer diabetischer Retinopathie, Birdshot-Chorioretinopathie, multifokaler Choroiditis und jeglicher Neovaskularisation, die mit jeglichem pathologischen Krankheitsbild des Auges verbunden ist.

## Revendications

1. Produit de combinaison comprenant une quantité thérapeutiquement efficace de (i) au moins un composé qui inhibe le VEGF et (ii) au moins un composé thrombolytique, dans lequel le composé qui inhibe le VEGF est le bévacizumab et le composé thrombolytique est l'activateur tissulaire du plasminogène (TPA).

2. Produit de combinaison pharmaceutique comprenant une quantité thérapeutiquement efficace de (i) au moins un composé qui inhibe le VEGF et (ii) au moins un composé thrombolytique, dans lequel le composé qui inhibe le VEGF est le bévacizumab et le composé thrombolytique est l'activateur tissulaire du plasminogène (TPA).

3. Produit de combinaison pharmaceutique selon la revendication 2 destiné à être utilisé dans le traitement des maladies néovasculaires du tissu oculaire chez un patient en ayant besoin.

4. Produit de combinaison pharmaceutique pour son utilisation selon la revendication 3, dans lequel ladite maladie néovasculaire est choisie dans le groupe constitué par l'oedème maculaire, la rétinopathie ischémique, la néovascularisation intraoculaire, la dégénérescence maculaire liée à l'âge, la néovascularisation cornéenne, la néovascularisation rétinienne, la néovascularisation choroïdale, la rétinopathie du prématuré, la lésion oculaire traumatique, l'oedème maculaire diabétique, l'ischémie rétinienne diabétique, l'oedème rétinien diabétique, la rétinopathie diabétique proliférative, la choriorétinopathie de type Birdshot, la choroïdite multifocale, et toute néovascularisation associée à un état pathologique quelconque de l'oeil.
